(19) Europäisches Patentamt

European Patent Office

Office européen des brevets

(11) **EP 2 505 121 B1**

(12) **EUROPEAN PATENT SPECIFICATION**

(45) Date of publication and mention
of the grant of the patent:
**19.03.2014 Bulletin 2014/12**

(21) Application number: **12161493.7**

(22) Date of filing: **27.03.2012**

(51) Int Cl.:
*A61B 1/06* (2006.01)      *A61B 1/00* (2006.01)

(54) **Endoscope apparatus**

Endoskopvorrichtung

Appareil d'endoscope

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.04.2011 JP 2011081438**

(43) Date of publication of application:
**03.10.2012 Bulletin 2012/40**

(73) Proprietor: **Fujifilm Corporation
Minato-ku
Tokyo (JP)**

(72) Inventors:
• **Minetoma, Yasuhiro
  Ashigara-kami-gun,
  Kanagawa (JP)**
• **Kaku, Toshihiko
  Ashigara-kami-gun,
  Kanagawa (JP)**

(74) Representative: **Klunker . Schmitt-Nilson . Hirsch
Patentanwälte
Destouchesstrasse 68
80796 München (DE)**

(56) References cited:
US-A1- 2008 214 940    US-A1- 2009 167 149
US-A1- 2011 071 352    US-A1- 2011 077 465

**Description**

BACKGROUND OF THE INVENTION

This invention relates to an endoscope apparatus capable of simultaneously acquiring blood vessel depth information and spectral estimation information.

In recent years, an endoscope apparatus capable of performing a so-called special light observation has been used so as to obtain tissue information at a desired depth of living body tissue by irradiating specific narrow wavelength band light (narrow band light) as illumination light to mucous tissue of a living body.

According to the special light observation, it is possible to simply visualize living body information which cannot be obtained in an ordinary observation image, such as emphasis of microscopic surface structures of new blood vessels generated in mucosa layers or submucosal layers and lesions. For example, when an observation subject is a carcinamatous lesion, the condition of microscopic blood vessels or microscopic structures of surface tissue may be more specifically observed by irradiating blue (B) narrow band light suitable for the observation of surface tissue and green (G) narrow band light suitable for the observation of intermediate tissue and surface tissue to mucous tissue. Accordingly, the lesions may be more accurately diagnosed.

Further, hitherto, an endoscope apparatus has been used so as to obtain a spectral estimation image with information on a predetermined wavelength band by performing spectral estimation based on a predetermined algorithm from a white image obtained by an ordinary light observation (see JP 4504324 B).

According to the spectral estimation image (the spectral image), the uneven shape of the mucous membranes or the degree of discoloration of tissue may be easily observed, and similar to the above, the lesions may be more accurately diagnosed. Further, in the case of a blood vessel depth image, for example, a blood vessel depth image in which surface blood vessel portions are emphasized, it is possible to more closely inspect the condition of surface blood vessels such as a brownish area useful for the diagnosis of carcinoma.

US 2011/071352 A1 discloses a device according to the preamble of claim 1.

SUMMARY OF THE INVENTION

However, in an endoscope apparatus which includes a light source device that irradiates quasi-white light by irradiating predetermined narrow band light from a narrow band light source and making a fluorescent substance emit fluorescent light, a B-light component, which is narrow band light, is strong and a B-image signal is saturated during the spectral estimation, so that it is difficult to simultaneously obtain blood vessel depth information and spectral estimation information, that is to say, to perform the spectral estimation while acquiring the blood vessel depth information.

Further, in the case of the above-described light source device, since the B-light component of the fluorescent light is added to the B-light component of the narrow band light also in the calculation of the blood vessel depth information, the blood vessel depth information is not easily calculated with high precision compared to the case where the blood vessel depth information is extracted by the separate narrow band light.

It is an object of the invention to provide an endoscope apparatus capable of more accurately diagnosing lesions in a manner such that high precision blood vessel depth information and spectral estimation information are simultaneously acquired and a blood vessel depth image and a spectral image are simultaneously generated and displayed.

In order to achieve the above object, the present invention provides an endoscope apparatus comprising: a light source device that includes a light source irradiating narrow band light having a predetermined wavelength bandwidth narrowed in relation to spectral characteristics of a structure and a component of a living body as a subject and a fluorescent substance excited by the narrow band light so as to emit predetermined fluorescent light and that irradiates illumination light including the narrow band light and the fluorescent light; an endoscope body that irradiates the illumination light toward the subject and that includes an imaging element capturing an image using returned light from the subject of the illumination light and outputting an image signal; and a processing device that includes a signal dividing unit dividing the image signal into a first image signal corresponding to the narrow band light and a second image signal corresponding to the fluorescent light, a blood vessel depth information calculating unit calculating blood vessel depth information based on the first image signal and the second image signal, a spectral estimation information calculating unit calculating spectral estimation information based on the second image signal, and an image processing unit generating a captured image from the first image signal, the second image signal, the blood vessel depth information, and the spectral estimation information.

It is preferable that the image signal includes a B-image signal, a G-image signal, and an R-image signal output in relation to the spectral sensitivity characteristics of the imaging element, and the signal dividing unit includes a signal estimating sub-unit and a calculation correction sub-unit, and divides the image signal into the first image signal corresponding to the narrow band light and the second image signal corresponding to the fluorescent light by using the signal estimating sub-unit to estimate the B-image signal corresponding to the fluorescent light from the G-image signal of the image signal, and using the calculation correction sub-unit to divide the B-image signal corresponding to the fluorescent light from the B-image signal of the image signal.

Preferably, the blood vessel depth information calculating unit includes a depth information table recording a correlation between a blood vessel depth and a ratio between the first image signal and the G-image signal (hereafter referred to as "$B_1$/G ratio"), and calculates the blood vessel depth information based on the $B_1$/G ratio and the depth information table.

The image processing unit preferably includes a blood vessel depth image generating sub-unit that generates a blood vessel depth image based on the $B_1$/G ratio.

It is preferable that the spectral estimation information is matrix information used for generating a spectral image signal from the second image signal, and the spectral estimation information calculating unit generates the spectral image signal from the second image signal by calculating the spectral estimation information.

The image processing unit preferably includes a spectral image generating sub-unit that generates a plurality of spectral images different from one another in wavelength band information from the spectral image signal.

Preferably, the spectral images as above are spectral images having different wavelength bands by 5 nm.

The light source is preferably a blue laser light source having a central emission wavelength of 445 nm.

It is preferable that the processing device further includes an ordinary light observation image generating unit that generates an ordinary light observation image based on the image signal, and a display device that displays an image generated by the processing device thereon.

It is preferable that a plurality of display devices, each identical to the above display device that displays an image generated by the processing device thereon, are provided, at least one special light observation image including the blood vessel depth image and the spectral image is displayed on at least one of the display devices, and the ordinary light observation image is displayed on at least one of the remaining display devices.

The processing device preferably includes a control unit that controls image display in the display device, whereupon it is preferable that, in the processing device, at least two display modes are set from a display mode in which only an ordinary light observation image is displayed on the display device, a display mode in which only a special light observation image is displayed on the display device, a display mode in which both an ordinary light observation image and a special light observation image, or both two different types of special light observation images are displayed on the display device in their entireties, a display mode in which both an ordinary light observation image and a special light observation image, or both two different types of special light observation images are displayed and a display range is changeable, and a display mode in which an ordinary light observation image and a special light observation image, or two different types of special light observation images are switched and displayed, and the processing device further includes a selection unit for making selection from the display modes.

According to the invention, since the blood vessel depth information and the spectral estimation information may be simultaneously acquired and the blood vessel depth image and the spectral image may be simultaneously generated and displayed in the endoscope observation, the lesions may be more accurately diagnosed.

BRIEF DESCRIPTION OF THE DRAWINGS

FIG. 1 is an external diagram illustrating an example of an endoscope apparatus according to the invention.
FIG. 2 is a block diagram conceptually illustrating a configuration of the endoscope apparatus shown in FIG. 1.
FIG. 3 is a conceptual diagram illustrating an example of a color filter of the imaging element shown in FIG. 2.
FIG. 4 is a graph illustrating a wavelength profile of the light source device shown in FIG. 2.
FIG. 5 is a graph illustrating the spectral sensitivity characteristics of the color filter of FIG. 3.
FIG. 6 is a graph illustrating a spectral reflectivity of a living body which is a test subject.
FIG. 7 is a block diagram conceptually illustrating a signal processing system of the endoscope apparatus shown in FIG. 1.
FIGS. 8A to 8C are diagrams illustrating a concept of an action of the signal dividing unit shown in FIG. 7.
FIG. 9 is a graph illustrating a blood vessel depth table which is provided in the blood vessel depth information calculating unit shown in FIG. 7.
FIG. 10 is a diagram illustrating a generation of a blood vessel depth image in the blood vessel depth image generating sub-unit of FIG. 7.

DETAILED DESCRIPTION OF THE INVENTION

An endoscope apparatus according to the invention will be described by referring to an exemplary embodiment shown in the accompanying drawings.

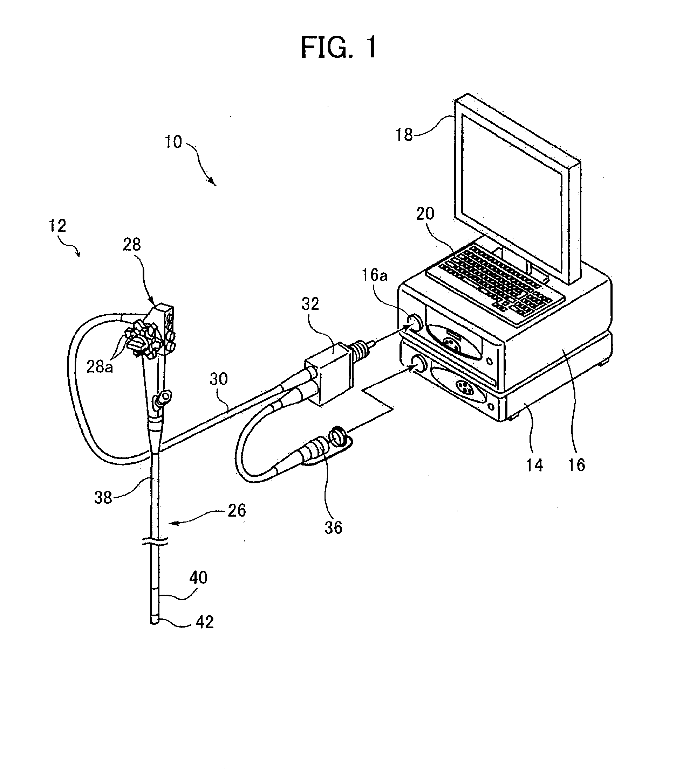

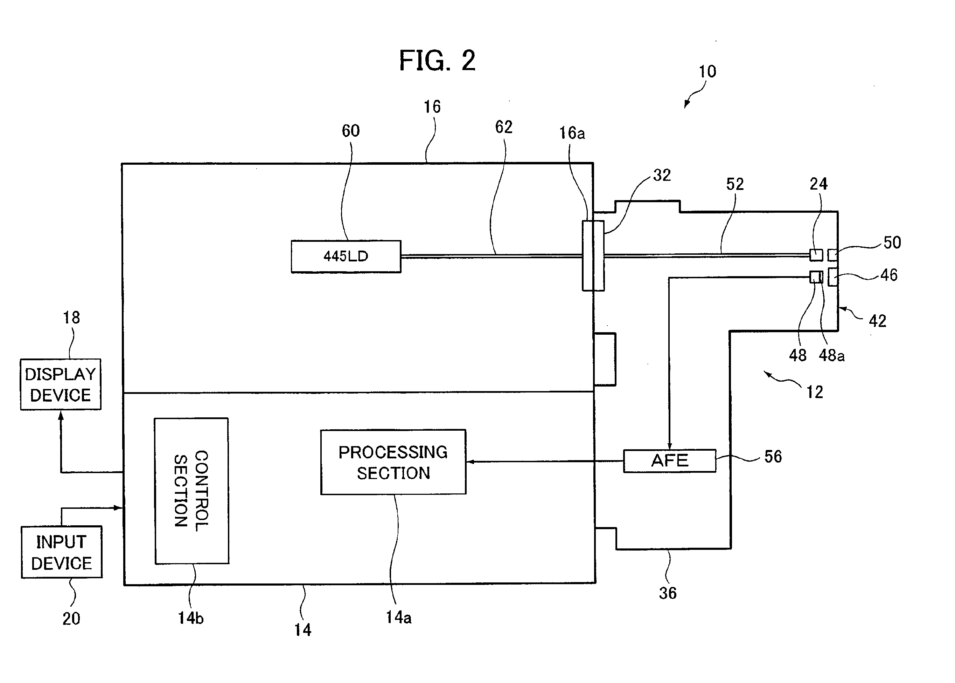

FIG. 1 is an external diagram illustrating a configuration of an endoscope apparatus 10 of the invention, and FIG. 2 is a block diagram conceptually illustrating a configuration of the endoscope apparatus shown in FIG. 1.

As shown in FIG. 1, as an example, the endoscope apparatus 10 includes: an endoscope 12; a processing device 14 which performs a process and the like on an image captured by the endoscope; and a light source device 16 which supplies illumination light used for the observation (the image capturing) in the endoscope 12. Further, the processing device 14 includes a display device 18 which displays an image captured by the endoscope and an input device 20 which inputs various commands, and the like (the display device 18 and the input device 20 are connected to the processing device 14).

Furthermore, the endoscope apparatus 10 of the invention may further include a printer (a recording device) which outputs the image captured by the endoscope as a hard copy.

As shown in FIG. 1, the endoscope 12 is an electronic endoscope which photoelectrically captures an image using an imaging element such as a CCD sensor 48. As in the case of an ordinary endoscope, the endoscope 12 includes an inserting unit 26, an operating unit 28, a universal cord 30, a connector 32, and a video connector 36.

In the endoscope 12, during an ordinary observation (diagnosis), the video connector 36 is connected to a connecting portion of the processing device 14 and the connector 32 is connected to a connecting portion 16a of the light source device 16. Furthermore, as in the case of the ordinary endoscope, a suction unit which suctions an observation portion or supplies air thereto, a water supply unit which sprays water to the observation portion, or the like is connected to the connector 32.

Further, as in the case of the ordinary endoscope, the inserting unit 26 of the endoscope 12 includes a base-end-side elongated flexible portion 38, a distal-end-side endoscopic portion (an endoscope distal end portion) 42 provided with the CCD sensor 48, or the like, and a curved portion (an angling portion) 40 disposed between the flexible portion 38 and the endoscopic portion 42. Furthermore, the operating unit 28 is provided with an operating knob 28a which curves the curved portion 40, or the like.

As conceptually shown in FIG. 2, the endoscopic portion 42 is provided with an imaging lens 46, a CCD sensor (an imaging element) 48, a color filter 48a, an illuminating lens 50, an optical fiber 52, and a cover glass (not shown) protecting the lenses, or the like.

Further, a fluorescent substance 24 which is a part of the light source device 16 is disposed at the distal end of the optical fiber 52. The fluorescent substance 24 comprises plural types of fluorescent substances (for example, YAG-based fluorescent substances or fluorescent substances such as BAM ($BaMgAl_{10}O_{17}$)) which absorb a part of B-light, and are excited to emit green to yellow light. Accordingly, the green to yellow fluorescent light using the B-light as the excitation light is mixed with the B-light transmitted without being absorbed to the fluorescent substance 24, so that quasi-white light is obtained.

Furthermore, although not shown in the drawings, the endoscope 12 is provided with a clamp channel and a clamp port through which various treatment instruments such as a clamp are inserted and an air-water supply channel and an air-water supply port through which a suctioning operation, an air supply operation, a water supply operation, and the like are performed.

The clamp channel communicates with a clamp inserting port provided in the operating unit 28 through the curved portion 40 and the flexible portion 38, and the air-water supply channel communicates with the connecting portion of the connector 32 connected to a suction unit, an air supply unit and a water supply unit, through the curved portion 40, the flexible portion 38, the operating unit 28, and the universal cord 30.

The optical fiber 52 is inserted to the connector 32 connected to the light source device 16 through the curved portion 40, the flexible portion 38, the operating unit 28, and the universal cord 30.

The narrow band light which is irradiated by the light source device 16 to be described later enters from the connector 32 into the optical fiber 52, is propagated through the optical fiber 52, and enters the fluorescent substance 24 installed before the distal end portion of the optical fiber 52 in the endoscopic portion 42 so that the fluorescent substance 24 is excited and fluorescent light is emitted therefrom. The fluorescent light emitted from the fluorescent substance 24, together with the narrow band light, enters the illuminating lens 50 and is irradiated as illumination light to an observation portion by the illuminating lens 50.

Furthermore, in the invention, the illumination light indicates any light which is irradiated to the observation portion regardless of if the light is narrow band light or fluorescent light.

Further, an image of an observation portion which is irradiated with the illumination light is formed on a light receiving surface of the CCD sensor 48 by the imaging lens 46.

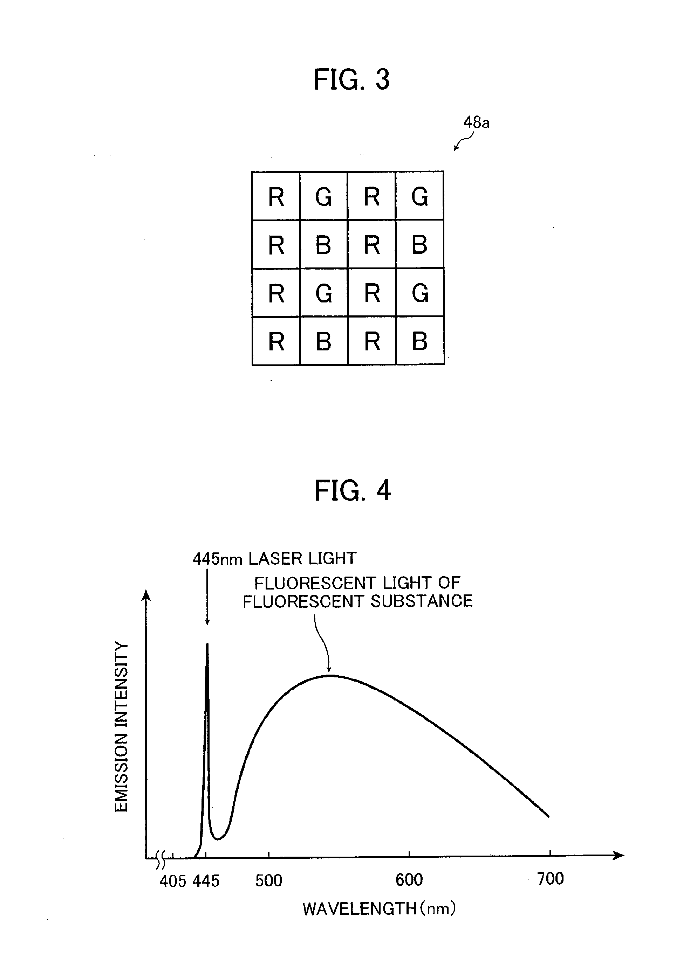

Here, in the invention, the CCD sensor 48 used in the endoscope 12 is a color CCD sensor in which a color filter 48a is equipped as shown in FIG. 2, and any one of a B (blue) filter, a G (green) filter, and an R (red) filter is provided for each pixel as shown in FIG. 3, to spectrally measure the incident light as B-light, G-light, and R-light. In other words, the CCD sensor 48 used in the endoscope 12 of the endoscope apparatus 10 of the invention is not a so-called frame-sequential type monochrome sensor which does not divide the incident light but sequentially measures the B-light, the G-light, and the R-light, but a synchronous color sensor which divides the incident light into the B-light, the G-light, and the R-light by the color filter 48a and measures each light synchronously.

Furthermore, in the invention, the imaging element is not limited to the CCD sensor 48, but various imaging elements such as a CMOS imaging sensor may be used as long as they are each a color sensor capable of dividing the light into the B-light, the G-light, and the R-light during the measurement of the light.

The output signal of the CCD sensor 48 is transmitted by a signal line from the endoscopic portion 42 to the video connector 36 through the curved portion 40, the flexible portion 38, the operating unit 28, the universal cord 30, and the connector 32.

In the example shown in the drawing, the video connector 36 is provided with an Analog Front End (AFE) substrate 56.

As an example, the AFE substrate 56 is provided with a correlated double sampling circuit, an amplifier (automatic gain control circuit), and an A/D converter. The output signal of the CCD sensor 48 undergoes a noise removing process using the correlated double sampling circuit and an amplifying process using the amplifier in the AFE substrate 56. Furthermore, the signal is converted from an analog signal into a digital signal by the A/D converter, and is output as a digital image signal to the processing device 14 (a digital signal processor 76 to be described later).

Furthermore, in the endoscope apparatus of the invention, these processes may be performed by not the video connector 36, but the connector 32 or the processing device 14.

As described above, in the endoscope apparatus 10, the connector 32 of the endoscope 12 is connected to the connecting portion 16a of the light source device 16.

The light source device 16 supplies illumination light which is used for inside observation of a living body to the endoscope 12. As described above, the narrow band light which is supplied from the light source device 16 to the endoscope 12 enters from the connector 32 into the optical fiber 52, is propagated through the optical fiber 52, and enters the fluorescent substance 24 installed before the distal end portion of the optical fiber 52 in the endoscopic portion 42 so that the fluorescent substance 24 is excited and fluorescent light is emitted therefrom. The fluorescent light emitted from the fluorescent substance 24, together with the narrow band light, enters the illuminating lens 50 and is irradiated as the illumination light to the observation portion by the illuminating lens 50.

As conceptually shown in FIG. 2, in the endoscope apparatus 10, the light source device 16 includes a light source 60, an optical fiber 62, the above-described connecting portion 16a, and the above-described fluorescent substance 24.

The light source 60 is a blue laser light source which irradiates narrow band light having a central wavelength of 445 nm, and excites the above-described fluorescent substance 24 so as to emit the fluorescent light, thereby irradiating quasi-white illumination light obtained by mixing the narrow band light and the fluorescent light to the observation portion.

FIG. 4 is a graph illustrating the emission spectrum of the light which is irradiated as the illumination light from the endoscope apparatus 10 of the invention to the observation portion. As described above, the light of FIG. 4 is mixed light which includes the blue laser light (the B-narrow-band light) having a central wavelength of 445 nm and the fluorescent light of the fluorescent substance.

The endoscope apparatus 10 of the invention uses the blue laser light and the fluorescent light as the illumination light, and simultaneously irradiates these lights to the observation portion and captures the image of the observation portion by using the CCD sensor 48 which divides the incident light into the B-light, the G-light, and the R-light during the measurement of the light.

Further, as described below in detail, the endoscope apparatus 10 (the processing device 14) generates an ordinary light observation image (an ordinary light image) by using the B-image, the G-image, and the R-image captured by the CCD sensor 48 of the endoscope 12, and also generates a special light observation image (a special light image) by using the B-image, the G-image, and the R-image captured by the CCD sensor 48. Here, the special light observation image indicates a blood vessel depth image and a spectral image (a narrow band light image) to be described later.

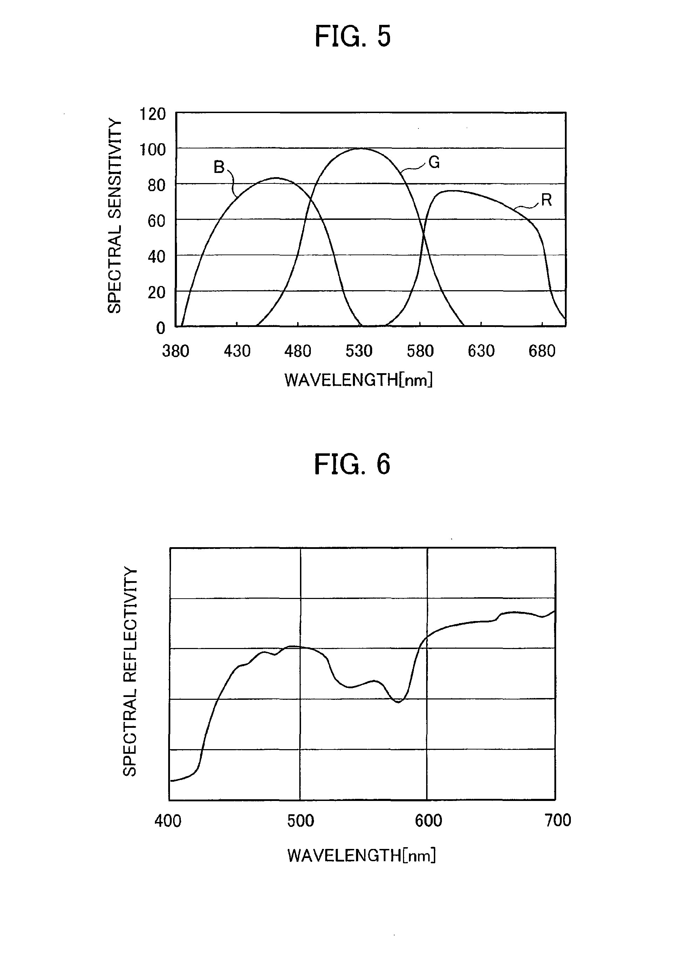

As conceptually shown in FIG. 5, in many cases, the pixels of respective colors of B, G, and R of the CCD sensor 48 have sensitivities even to the regions of the adjacent colors due to the filter characteristics for respective colors of B, G, and R (the color filter characteristics).

That is, not only the G-band light but also the R-band light (or a part thereof) enter the G-pixel, and are measured. Further, not only the B-narrow-band light (the blue laser light) but also the G-band light (or a part thereof) enter the B-pixel, and are measured.

In this regard, for example, when the B-band light amount is set to be larger than the G-band light amount, the B-band light may be made dominant in the B-band light and the G-band light which enter the B-pixel of the CCD sensor 48. In the same way, when the G-band light amount is set to be larger than the R-band light amount, the G-band light may be made dominant in the G-band light and the R-band light which enter the G-pixel of the CCD sensor 48.

Since the light source device 16 of the invention comprises the blue laser light source which serves as the light source 60 and the fluorescent substance 24 which emits the fluorescent light through the excitation by the blue laser light, the above-described configuration (B-light>G-light and G-light>R-light) is provided.

With such a configuration (B-light>G-light and G-light>R-light), it is possible to generate an appropriate ordinary light observation image from the image which is read out by the CCD sensor 48.

With regard to the light amount ratio between the B-band light and the G-band light and the light amount ratio between the G-band light and the R-band light in the invention, since the fluorescent substance and the blue laser light source serving as the light source are used, the G-band light and the R-band light which are each fluorescent light are in a relationship where they are determined in amount according to the light amount of the blue laser light, so that the light amount ratios are also determined according to the light amount of the blue laser light. The light amount of the blue laser light is adjusted by a light amount adjustment unit (not shown) which is controlled by a control section 14b to be described later.

The light which is supplied to the connecting portion 16a of the light source device 16 is supplied to the connector 32 of the endoscope 12, enters from the connector 32 into the optical fiber 52 so as to be propagated therethrough, and is irradiated as illumination light from the endoscopic portion 42 of the endoscope 12 to the observation portion.

The irradiated illumination light is reflected from the living body based on the spectral reflectivity thereof shown in FIG. 6, enters the imaging lens 46, and forms an image on the image receiving surface of the CCD sensor 48.

The image of the observation portion irradiated with the illumination light is captured by the CCD sensor 48. As described above, the image which is captured by the CCD sensor 48 (the output signal of the CCD sensor 48) undergoes a process such as an A/D conversion process by the AFE substrate 56, and the output is supplied as a digital image signal (image data/image information) to the processing device 14.

The processing device 14 controls the entire endoscope apparatus 10 and performs a predetermined process on the image signal supplied (output) from the endoscope 12 so as to display the image captured by the endoscope 12 on the display device 18, and the device 14 has an image signal processing section 14a and the control section 14b controlling the entire processing device 14 and the entire endoscope apparatus 10.

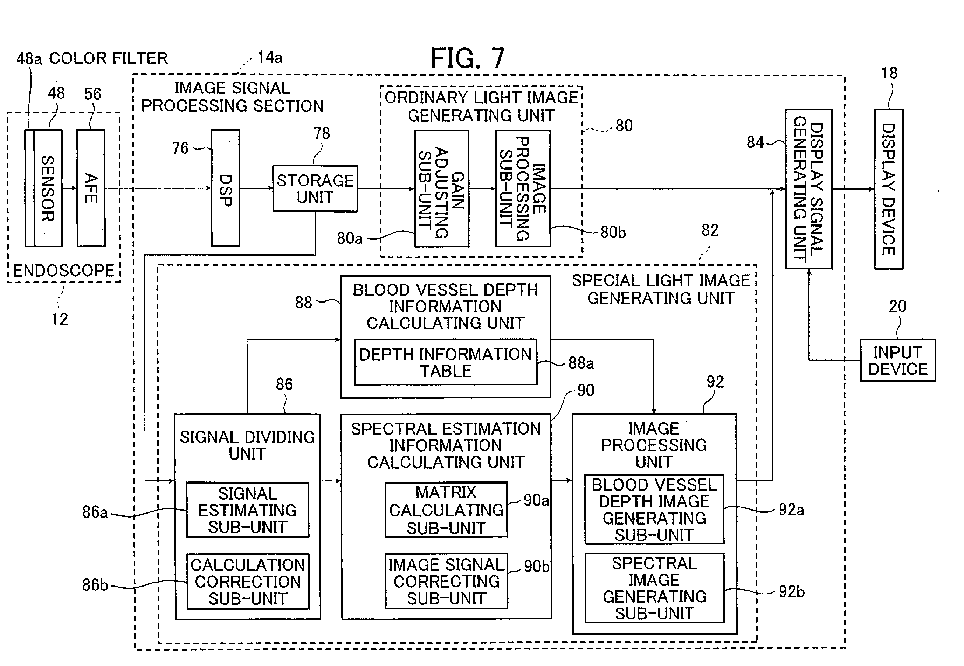

FIG. 7 is a block diagram conceptually illustrating the image signal processing section 14a of the processing device 14.

As shown in FIG. 7, the processing section 14a includes the digital signal processor (DSP) 76, a storage unit 78, an ordinary light image generating unit 80, a special light image generating unit 82, and a display signal generating unit 84.

In the processing device 14, a predetermined process such as a gamma correction process and a color correction process is first performed by the DSP 76 on the image signal (the B-image signal, the G-image signal, and the R-image signal) of the image captured by the CCD sensor 48 and processed by the AFE 56, and then the image signal is stored in the storage unit (the memory) 78.

When the image signal is stored in the storage unit 78, the ordinary light image generating unit 80 reads the image signals of B, G, and R from the storage unit 78, and generates the ordinary light observation image. Furthermore, in the same way, the special light image generating unit 82 reads the image signals of B, G, and R from the storage unit 78, and generates the special light observation image.

Furthermore, when there is a command for the generation (display) only for the ordinary light observation image or the special light observation image in advance through the input device 20 or the like, only the image generating unit which receives the command for the generation may read the image signal from the storage unit 78 and may perform a process to be described later.

As described above, in the endoscope apparatus 10, the illumination light supplied from the light source device 16 and shown in FIG. 4 is irradiated, that is, both the blue laser light and the fluorescent light are simultaneously irradiated to the observation portion. Further, the image of the observation portion is captured by the CCD sensor 48 which divides the incident light into the B-light, the G-light, and the R-light during the measurement of the incident light.

That is, in the endoscope apparatus 10 of the invention, the quasi-white light which is obtained by mixing the blue laser light and the fluorescent light is used as the illumination light, and the image of observation portion is captured by the color CCD sensor 48 which measures the B-light, the G-light, and the R-light (the light components of respective colors) of the incident light.

Thus, when the display image is generated by using the B-image signal, the G-image signal, and the R-image signal measured by the CCD sensor 48, it is possible to generate the image of the ordinary light observation using the white light, that is, the ordinary light as the illumination light.

Further, as described below, when the display image is generated by using the B-image signal and the G-image signal measured by the CCD sensor 48, it is possible to generate the image of the special light observation.

That is, according to the endoscope apparatus of the invention, it is possible to simultaneously obtain the ordinary light observation image and the special light observation image from one captured image without the switching time lag between the ordinary light observation and the special light observation by using the basic configuration of the general endoscope apparatus (the endoscope system).

The ordinary light image generating unit 80 includes a gain adjusting sub-unit 80a and an image processing sub-unit 80b.

As a desirable configuration, the gain adjusting sub-unit 80a adjusts the gain of the image signals of B, G, and R read out from the storage unit 78 and obtains the same image signal as that of the observation using the ordinary white light.

The gain adjusting sub-unit 80a performs a gain adjusting process on the image signals of B, G, and R, for example, an amplifying process on the image signals of G and R or a restriction process on the image signals of B and G so that the image signal is the same as that of the case where the image is captured with the white illumination light having equal light amounts of B, G, and R.

The gain adjusting method is not particularly limited, and various methods may be used if the light amount difference of B, G, and R of the illumination light is balanced so that the image signal (the image captured by the CCD sensor 48) is the same as that of the case where the image is captured with the illumination light having uniform light amounts of B, G, and R.

As an example, a method may be used in which the correction coefficient that is created for balancing the light amount difference between respective lights in accordance with the light amount difference (the light amount ratio) between B and G and the light amount difference between G and R is multiplied or added, or divided or subtracted with respect to the respective image signals. Further, a method may be used in which the respective image signals are processed by using the LUT that is created so as to balance the light amount difference between the respective lights in accordance with the light amount difference between B and G and the light amount difference between G and R.

The image processing sub-unit 80b performs a color conversion process such as a three-by-three matrix process, a grayscale conversion process, and a three-dimensional LUT process; a color emphasizing process for making a difference in the hue between the blood vessel and the mucous membrane in an image more distinct against the average hue of the image so that the blood vessel is easily seen through a difference in the hue between the blood vessel and the mucous membrane on the screen; an image structure emphasizing process such as a sharpness process or an edge emphasizing process; and the like on the image signal subjected to the gain adjusting process, and the output is supplied as the image signal of the ordinary light observation image to the display signal generating unit 84.

On the other hand, the special light image generating unit 82 includes a signal dividing unit 86, a blood vessel depth information calculating unit 88, a spectral estimation information calculating unit 90, and an image processing unit 92.

As a desirable configuration, the signal dividing unit 86 includes a signal estimating sub-unit 86a and a calculation correction sub-unit 86b, and divides the image signal into a first image signal based on the blue laser light and a second image signal based on the fluorescent light. Specifically, since the blue laser light is only the B-light component and the reflected light from the living body is also the B-light component, the B-image signal of the image signal is divided into a first B-image signal based on the blue laser light and a second B-image signal based on the fluorescent light.

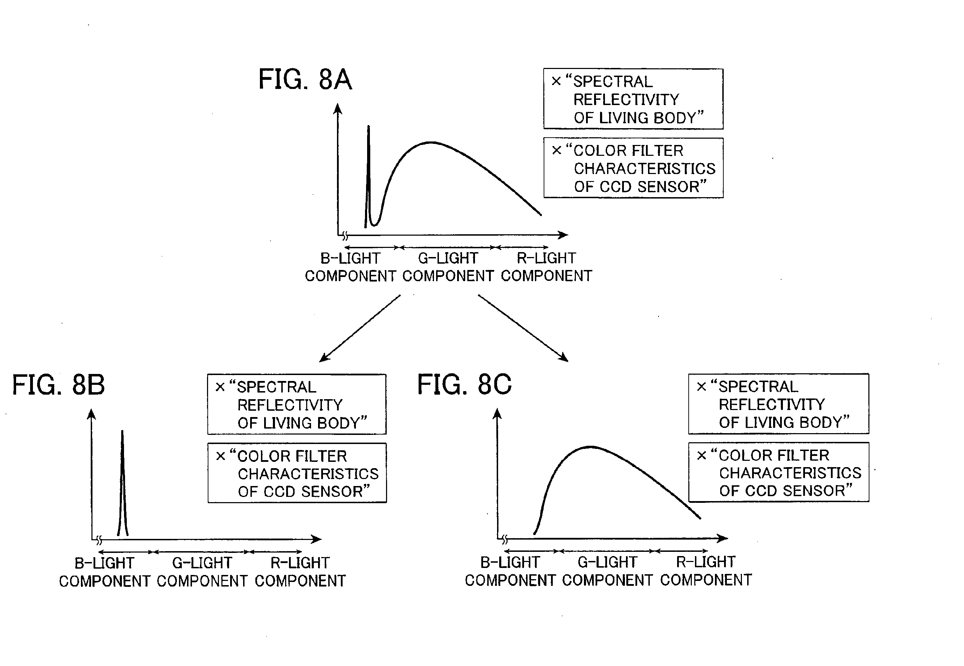

FIGS. 8A to 8C are diagrams illustrating a concept in which the signal dividing unit 86 divides the image signal (FIG. 8A) into the first image signal (FIG. 8B) and the second image signal (FIG. 8C). Furthermore, as shown in FIG. 8A, in fact, the signal which is obtained by multiplying the spectroscopic profile of the illumination light of FIG. 4 by the "color filter characteristics of the CCD sensor" shown in FIG. 5 and the "spectral reflectivity of the living body" shown in FIG. 6 is measured by the CCD sensor 48, and is output to the signal dividing unit 86.

The signal dividing unit 86 divides the output image signal into the image signal based on the narrow band light and the image signal based on the fluorescent light. Since the "spectral reflectivity of the living body" and the "color filter characteristics of the CCD sensor" are common to the two image signals, for convenience of description, as shown in FIGS. 8A to 8C, the division of the image signal is described by referring to the spectroscopic profile of the illumination light.

Specifically, first, in the signal dividing unit 86, the value of the second B-image signal as the B-image signal based on the fluorescent light is estimated from the value of the G-image signal in the image signal using the signal estimating sub-unit 86a.

Now, as described above, the spectroscopic profile of the illumination light has a shape shown in FIG. 4, and the emission intensity of the illumination light changes with the irradiation light amount (the emission intensity) of the laser light of 445 nm as the excitation light. However, since the general shape of the spectroscopic profile is substantially unchanged, it is possible to estimate the B-light component of the fluorescent light, that is, the second B-image signal from the G-light component of the fluorescent light, that is, the G-light image signal of the image signal.

The signal dividing unit 86 calculates the first B-image signal by dividing the second B-image signal from the B-image signal of the image signal in the calculation correction sub-unit 86b using the second B-image signal estimated by the signal estimating sub-unit 86a. The calculated first B-image signal is the first image signal, and the second B-image signal, the G-image signal, and the R-image signal are the second image signal.

In this way, the signal dividing unit 86 divides the image signal read out from the storage unit 78 into the first image signal based on the blue laser light and the second image signal based on the fluorescent light. The first image signal and the second image signal which are divided from each other are supplied to the blood vessel depth information calculating unit 88 and the spectral estimation information calculating unit 90, respectively.

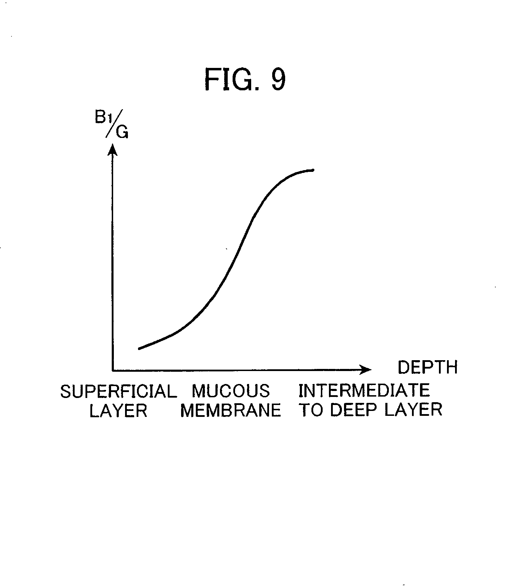

The blood vessel depth information calculating unit 88 calculates a $B_1/G$ ratio which is a ratio between the first B-image signal as the acquired first image signal and the G-image signal in the second image signal, and calculates the blood vessel depth information from the calculated $B_1/G$ ratio and a depth information table 88a shown in FIG. 9.

The blood vessel depth information is output to the image processing unit 92 together with the $B_1/G$ ratio.

Further, the spectral estimation information calculating unit 90 includes a matrix calculating sub-unit 90a and an image signal correcting sub-unit 90b, calculates the matrix as the spectral estimation information from the image signal (the second image signal), and generates the spectral image signal.

Since the generation of the spectral image signal is generally known as disclosed in JP 4504324 B, hereinafter, the generation will be simply described.

The matrix calculating sub-unit 90a calculates a matrix which is a predetermined coefficient that is used to generate the spectral image signal from the second image signal as the color image signal.

The matrix is calculated as, for example, n-dimensional column vectors "R", "G", and "B" by changing the color sensitivity characteristics as the spectral sensitivity characteristics of the CCD sensor 48 as the imaging element into numerical data.

Next, n-dimensional column vectors "F1", "F2", and "F3" are obtained by changing the characteristics of narrow-band pass filters F1, F2, and F3 for the spectral image as the basic spectral characteristics of the spectral signals to be extracted, for example, three spectral signals into numerical values.

Then, the optimal coefficient set which is approximate to the relationship of the following equation (1) is obtained based on the obtained numerical values. That is, the matrix "A" may be obtained which satisfies the following equation.

$$(R, \ G, \ B) \cdot A = (F1, \ F2, \ F3) \ \ldots \ (1)$$

The method of calculating the matrix "A" is generally known as described above, and the spectral estimation information-calculating unit 90 generates the spectral image signal from the second image signal using the calculated matrix "A".

Further, the spectral estimation information calculating unit 90 includes the image signal correcting sub-unit 90b which calculates the more accurate matrix (the spectral estimation information) and obtains the accurate spectral image signal.

The above-described matrix calculating sub-unit 90a is accurately applied and is made optimal for approximation when the light flux received by the CCD sensor 48 is the perfect white light (that is, when the R-, G- and B-outputs are the same).

However, since the illuminating light flux (the light flux of the light source) is not the perfect white light and the reflection spectrum (the spectral reflectivity) of the living body is not uniform under the actual endoscope observation, the light flux which is received by the solid-state imaging element is not the white light.

Thus, it is desirable to consider the spectral sensitivity characteristics (the color filter characteristics) of the CCD sensor 48, the spectral sensitivity characteristics of the illumination light, and the spectral reflectivity of the living body in the actual process. Thus, in the image signal correcting sub-unit 90b, the signal correction based on these characteristics is performed on the second image signal. For this correction, as described above, the known correction method disclosed in JP 4504324 B is used.

The spectral image signals of R, G, and B calculated from the second image signal are output to the image processing unit 92.

The image processing unit 92 includes a blood vessel depth image generating sub-unit 92a and a spectral image generating sub-unit 92b, and generates the blood vessel depth image from the blood vessel depth information and the $B_1/G$ ratio and the spectral image from the spectral image signal.

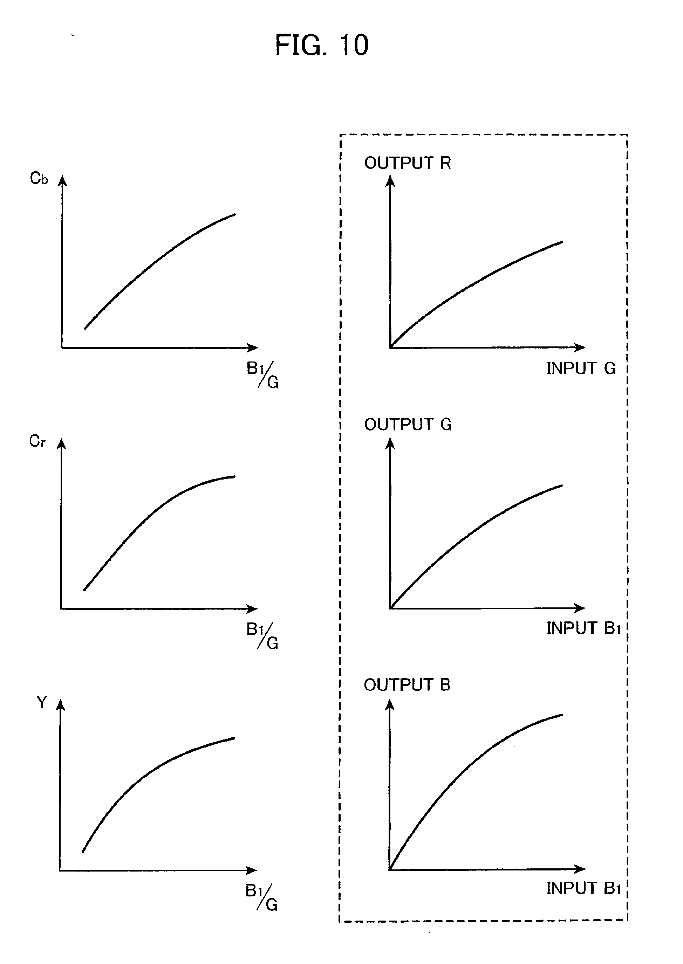

The generation of the blood vessel depth image in the blood vessel depth image generating sub-unit 92a is performed by generating the blood vessel depth image based on the $B_1/G$ ratio. For example, the generation of the blood vessel depth image is performed in a manner such that the luminance signal Y and the color difference signals Cr and Cb (hereinafter, referred to as a YCC signal) are calculated based on the $B_1/G$ ratio of the pixel, as shown in FIG. 10 and the calculated YCC signal is converted into RGB-image signals again. This is the same as the signal conversion which outputs predetermined RGB-image signals with respect to the input of the predetermined $B_1$-image signal and the predetermined G-image signal as depicted by the dotted line in FIG. 10.

Based on the signal conversion, the blood vessel depth image is generated in the blood vessel depth image generating sub-unit 92a. Furthermore, the blood vessel depth information which is calculated in the blood vessel depth information calculating unit 88 may be correlated to the generated blood vessel depth image. By the correlation between the blood vessel depth image and the blood vessel depth information, it is possible to determine the degree of the blood vessel depth in a predetermined region of the image.

The generated blood vessel depth image and the correlated blood vessel depth information are output to the display signal generating unit 84.

The spectral image generating sub-unit 92b generates the spectral image based on the spectral image signal from the spectral estimation information calculating unit 90.

The spectral image may be generated by allocating a predetermined spectral image signal to the respective image signals of R, G, and B. The spectral image signal may be calculated by, for example, 5 nm, and plural spectral images are generated which have different wavelength bands by 5 nm in the spectral image generating sub-unit 92b. The generated plural spectral images are output to the display signal generating unit 84.

In addition, the image processing unit 92 performs a color conversion process such as a three-by-three matrix process, a grayscale conversion process, and a three-dimensional LUT process; a color emphasizing process for making a difference in the hue between the blood vessel and the mucous membrane in an image more distinct against the average hue of the image so that the blood vessel is easily seen through a difference in the hue between the blood vessel and the mucous membrane on the screen; an image structure emphasizing process such as a sharpness process or an edge emphasizing process; and the like on the respective image signals so that the output is supplied as the image signal of the special light observation image (the blood vessel depth image and the spectral image) to the display signal generating unit 84.

The display signal generating unit 84 performs a necessary process such as a color space conversion process on the supplied image signal of the ordinary light observation image and the supplied image signal of the special light observation image so as to obtain an image signal for the display by the display device 18.

Here, in the endoscope apparatus 10, as an example, at least two display modes are set from the display mode in which only an ordinary light observation image is displayed; the display mode in which only a special light observation image (blood vessel depth image or spectral estimation image) is displayed; the display mode in which the entirety of an ordinary light observation image and the entirety of a special light observation image, or the entireties of two different types of special light observation images (including a blood vessel depth image and a spectral estimation image) are arranged and displayed with sizes fit for one screen of the display device 18 (the sizes of two images may be equal to or different from each other, or adjustable); the display mode in which an ordinary light observation image and a special light observation image, or two different types of special light observation images are arranged and displayed with sizes over one screen of the display device 18, and the display range is changeable by a slider bar, a track ball, or the like; and the display mode in which an ordinary light observation image and a special light observation image, or two different types of special light observation images are switched and displayed in accordance with the switching command from the input device 20 and/or the switching unit set in the operating unit 28 of the endoscope 12 (the switching display includes the switching of the wavelength band of a spectral estimation image).

Further, these display modes may be selected and commanded through the input device 20 and/or the selection unit set in the operating unit 28 of the endoscope 12.

The display signal generating unit 84 generates a display image signal by performing an image size increasing-decreasing process and an image layout process in accordance with the selected display mode, and further performing an adding process with character information such as a test subject's name, and displays the image based on the display image signal on the display device 18.

Further, the numerical display of the blood vessel depth information, the numerical display of the corresponding wavelength band in the spectral image, and the like are added to the display image signal in the display signal generating unit 84.

Further, when plural display devices 18 are provided, the display signal generating unit 84 may generate the image signals so that the ordinary light observation image is displayed on one display device 18 and the special light observation image is displayed on another display device 18.

Alternatively, when three or more display devices 18 are provided, the ordinary light observation image and the special light observation image may be displayed on different display devices as described above, and the image according to any of the display modes may be displayed on yet another display device.

Further, the display signal generating unit 84 selects the spectral image and generates the display image based on the wavelength band of the spectral image to be displayed according to the command from the input device 20.

Further, the spectral image which is displayed on the display device 18 may gradually change the corresponding wavelength band according to the command from the input device 20.

Hereinafter, an example of an operation of the endoscope apparatus 10 will be described.

When the image capturing operation using the endoscope 12 is started by the command from the input device 20, the light source 60 of the light source device 16 is turned on, the illumination light with a predetermined light amount is irradiated to the subject, and then the CCD sensor 48 starts to capture an image (measure light).

The blue laser light (the narrow band light) which is irradiated by the light source 60 passes through an optical fiber 62, is then supplied from the connecting portion 16a to the connector 32 of the endoscope 12, and propagated to the endoscopic portion 42 at the tip through the optical fiber 52. The propagated blue laser light excites the fluorescent substance 24 which is installed at the distal end of the optical fiber 52 so as to generate the fluorescent light, and is irradiated as the illumination light including the blue laser light and the fluorescent light to the observation portion (inside of the living body) by the illuminating lens 50.

The image of the observation portion irradiated with the illumination light is formed on the light receiving surface of the CCD sensor 48 by the imaging lens 46, and is captured (subjected to light measurement) by the CCD sensor 48.

The output signal of the CCD sensor 48 is supplied to the AFE substrate 56. The AFE substrate 56 performs a noise removing process using the correlated double sampling, an amplifying process, an A/D conversion process, and the like on the output signal of the CCD sensor 48, and the output is supplied as the digital image signal to the DSP 76 of the processing device 14 (the image signal processing section 14a).

The DSP 76 performs a predetermined process such as a gamma correction and a color correction process on the image signal, and stores the processed image signal in the storage unit 78.

When the image signal is stored in the storage unit 78, the ordinary light image generating unit 80 and the special light image generating unit 82 respectively read image signals of B, G and R from the storage unit 78.

In the ordinary light image generating unit 80, the gain adjusting sub-unit 80a performs a gain adjusting process on the image signal read out to obtain the same image as the image captured with the white light having equal light amounts of B, G, and R as described above. Furthermore, the image processing sub-unit 80b performs a color conversion process, a color emphasizing process, and an image structure emphasizing process on the image signal subjected to the gain adjusting process, so that the output is supplied as the image signal of the ordinary light observation image to the display signal generating unit 84.

On the other hand, in the special light image generating unit 82, the signal dividing unit 86 divides the read image signal into the first image signal (the first B-image signal) and the second image signal (the second B-image signal, the G-image signal, and the R-image signal), and outputs the signals to the blood vessel depth information calculating unit 88 and the spectral estimation information calculating unit 90.

The blood vessel depth information calculating unit 88 calculates the $B_1/G$ ratio as the ratio between the first B-image signal and the G-image signal, and calculates the blood vessel depth information based on the $B_1/G$ ratio and the depth information table 88a. The calculated $B_1/G$ ratio and the calculated blood vessel depth information are output to the blood vessel depth image generating sub-unit 92a of the image processing unit 92.

Further, in the spectral estimation information calculating unit 90, the spectral estimation information (the matrix) is calculated by the matrix calculating sub-unit 90a and the image signal correcting sub-unit 90b so as to generate the spectral image signal. The generated spectral image signal is output to the spectral image generating sub-unit 92b of the image processing unit 92.

The image processing unit 92 generates the blood vessel depth image as shown in FIG. 10 using the blood vessel depth image generating sub-unit 92a based on the $B_1/G$ ratio and the blood vessel depth information. Further, the image processing unit 92 generates the spectral image using the spectral image generating sub-unit 92b based on the spectral image signal.

Furthermore, the image processing unit 92 performs a color conversion process, a color emphasizing process, and an image structure emphasizing process on the image signal, so that the output is supplied as the image signal of the special light observation image including the blood vessel depth image and the spectral image to the display signal generating unit 84.

The display signal generating unit 84 to which the image signals of the ordinary light observation image and the special light observation image are supplied generates the display image signal in accordance with the display mode which is selected and commanded through the input device 20, for example, an image signal for arranging and displaying the entireties of the ordinary light observation image and the special light observation image on one screen of the display device 18, and displays the image based on the display image signal on the display device 18.

Further, the displayed spectral image may be changed by inputting the corresponding wavelength band of the spectral image from the input device 20.

The above-described operation is the operation of the endoscope apparatus 10 according to the embodiment of the invention.

Further, in the endoscope apparatus 10 of the invention, the special light observation image may be generated by directly using the B-image signal and the G-image signal.

In this case, as described above, in the image which is captured by the CCD sensor 48, not only the G-narrow-band light but also the R-band light enter the G-pixel and are measured, and not only the B-narrow-band light but also the G-narrow-band light enter the B-pixel and are measured, due to the color filter characteristics of the sensor 48. Thus, when the special light observation image is generated by directly using the B-image signal and the G-image signal, an image is obtained in which the B-image is affected by the G-image component and the G-image is affected by the R-image component.

For this reason, it is desirable that the signal dividing unit 86 eliminates the R-image signal component from the G-image signal by processing the G-image signal using the R-image signal and eliminates the G-image signal component from the B-image signal by processing the B-image signal using the G-image signal.

Furthermore, as the R-image signal used for the process of the G-image signal and the G-image signal used for the process of the B-image signal, image signals of the R-sub-pixel and the G-sub-pixel forming one pixel together with the pixel to be processed may be used, respectively. Alternatively, a pixel adjacent to the pixel to be processed is appropriately selected, and the image signal of the selected pixel may be used.

As an example, the correction of the G-image signal is performed by the following equation.

```
corrected G-image signal = G-image signal - α × R-image
```

```
signal
```

(that is, corrected G-pixel = G-pixel - α x R-pixel)

Here, $\alpha$ denotes the coefficient used for obtaining the R-light component measured in the G-pixel, and in accordance with the color filter characteristics or the like of the CCD sensor 48, the coefficient used for calculating the R-light component measured in the G-pixel may be appropriately set.

The corrected G-image signal may be used instead of the G-image signal to obtain the $B_1/G$ ratio which is used to calculate the blood vessel depth information and the blood vessel depth image.

While the endoscope apparatus of the invention has been described above in detail, the invention is not limited to the above-described embodiment and its scope is defined by the appended claims.


**Claims**

1.  An endoscope (12) apparatus comprising:

    a light source device (16) that includes a light source (60) irradiating narrow band light having a predetermined wavelength bandwidth narrowed in relation to spectral characteristics of a structure and a component of a living body as a subject and a fluorescent substance (24) excited by the narrow band light so as to emit predetermined fluorescent light and that irradiates illumination light including the narrow band light and the fluorescent light;
    an endoscope body that irradiates the illumination light toward the subject and that includes an imaging element (48) capturing an image using returned light from the subject of the illumination light and outputting an image signal; and
    a processing device (14); **characterized by** the processing device including a signal dividing unit (86) dividing the image signal into a first image signal corresponding to the narrow band light and a second image signal corresponding to the fluorescent light, a blood vessel depth information calculating unit (88) calculating blood vessel depth information based on the first image signal and the second image signal, a spectral estimation information calculating unit (90) calculating spectral estimation information based on the second image signal, and an image processing unit (92) generating a captured image from the first image signal, the second image signal, the blood vessel depth information, and the spectral estimation information.

2.  The endoscope apparatus according to claim 1,
    wherein the image signal includes a B-image signal, a G-image signal, and an R-image signal output in relation to spectral sensitivity characteristics of the imaging element,
    wherein the signal dividing unit includes a signal estimating means and a calculation correction means, and
    wherein the signal estimating means estimates the B-image signal corresponding to the fluorescent light from the G-image signal of the image signal, and the calculation correction means divides the B-image signal corresponding to the fluorescent light from the B-image signal of the image signal, so that the image signal is divided into the first image signal corresponding to the narrow band light and the second image signal corresponding to the fluorescent light by the signal dividing unit.

3.  The endoscope apparatus according to claim 2,
    wherein the blood vessel depth information calculating unit includes a depth information table recording a correlation between a blood vessel depth and a ratio between the first image signal and the G-image signal, with the ratio being expressed as $B_1/G$, and
    wherein the blood vessel depth information calculating unit calculates the blood vessel depth information based on the $B_1/G$ ratio and the depth information table.

**4.** The endoscope apparatus according to claim 3,
wherein the image processing unit includes a blood vessel depth image generating means that generates a blood vessel depth image based on the $B_1$/G ratio.

**5.** The endoscope apparatus according to any one of claims 1 to 4,
wherein the spectral estimation information is matrix information used for generating a spectral image signal from the second image signal, and
wherein the spectral estimation information calculating unit generates the spectral image signal from the second image signal by calculating the spectral estimation information.

**6.** The endoscope apparatus according to claim 5,
wherein the image processing unit includes a spectral image generating means that generates a plurality of spectral images different from one another in wavelength band information from the spectral image signal.

**7.** The endoscope apparatus according to claim 6,
wherein the plurality of spectral images are spectral images having different wavelength bands by 5 nm.

**8.** The endoscope apparatus according to any one of claims 1 to 7,
wherein the light source is a blue laser light source having a central emission wavelength of 445 nm.

**9.** The endoscope apparatus according to any one of claims 1 to 8,
wherein the processing device further includes an ordinary light observation image generating unit that generates an ordinary light observation image based on the image signal, and a display device that displays an image generated by the processing device thereon.

**10.** The endoscope apparatus according to claim 9,
wherein a plurality of display devices, each identical to the display device that displays an image generated by the processing device thereon, are provided, at least one special light observation image including the blood vessel depth image and the spectral image is displayed on at least one of the display devices, and the ordinary light observation image is displayed on at least one of the remaining display devices.

**11.** The endoscope apparatus according to claim 9 or 10,
wherein the processing device includes a control unit that controls image display in the display device,
wherein in the processing device, at least two display modes are set from a display mode in which only an ordinary light observation image is displayed on the display device, a display mode in which only a special light observation image is displayed on the display device, a display mode in which both an ordinary light observation image and a special light observation image, or both two different types of special light observation images are displayed on the display device in their entireties, a display mode in which both an ordinary light observation image and a special light observation image, or both two different types of special light observation images are displayed and a display range is changeable, and a display mode in which an ordinary light observation image and a special light observation image, or two different types of special light observation images are switched and displayed, and
wherein the processing device further includes a selection means for making selection from the display modes.

**Patentansprüche**

**1.** Endoskopvorrichtung (12), umfassend:

eine Lichtquelleneinrichtung (16), die eine Lichtquelle (60) enthält, welche schmalbandiges Licht mit einer vorbestimmten Wellenlängenbandbreite, verschmälert in Bezug auf Spektralkennlinien einer Struktur und einer Komponente eines lebenden Körpers als Subjekt und einer von dem schmalbandigen Licht angeregten fluoreszierenden Substanz (24) strahlt, um vorbestimmtes Fluoreszenzlicht zu emittieren, und die Beleuchtungslicht abstrahlt, welches das schmalbandige Licht und das Fluoreszenzlicht enthält;
einen Endoskopkörper, der das Beleuchtungslicht in Richtung des Subjekts abstrahlt, und der ein Abbildungselement (48) enthält, welches ein Bild aufnimmt unter Verwendung von aus dem Subjekt zurückgeworfenen Licht des Beleuchtungslichts, und zum Ausgeben eines Bildsignals; und
eine Verarbeitungseinrichtung (14); **dadurch gekennzeichnet, dass** die Verarbeitungseinrichtung eine Signalteilereinheit (86) enthält, die das Bildsignal aufteilt in ein erstes Bildsignal entsprechend dem schmalbandigen Licht und ein zweites Bildsignal entsprechend dem Fluoreszenzlicht, das eine Blutgefäßtiefeninformations-Berechnungseinrichtung (88) Blutgefäßtiefeninformation basierend auf dem ersten Bildsignal und dem zweiten Bildsignal berechnet, eine Spektralabschätzinformations-Berechnungseinheit (90) Spektralabschätzinformation basierend auf dem zweiten Bildsignal berechnet, und dass eine Bildverarbeitungseinheit (92) ein aufgenommenes Bild aus dem ersten Bildsignal, dem zweiten Bildsignal, der Blutgefäßtiefeninformation und der Spektralabschätzinformation erzeugt.

2. Endoskopvorrichtung nach Anspruch 1,
bei der das Bildsignal ein B-Bildsignal, ein G-Bildsignal und ein R-Bildsignal enthält, die in Bezug auf die Spektral-Empfindlichkeitskennlinie des Abbildungselements ausgegeben werden,
wobei die Signalteilereinheit eine Signalabschätzeinrichtung und eine Berechnungskorrektureinrichtung enthält, und
wobei die Signalabschätzeinrichtung das B-Bildsignal entsprechend dem Fluoreszenzlicht aus dem G-Bildsignal des Bildsignals abschätzt und die Berechnungskorrektureinrichtung das B-Bildsignal entsprechend dem Fluoreszenzlicht aus dem B-Bildsignal des Bildsignals abteilt, so dass das Bildsignal von der Signalteilereinheit aufgeteilt wird in das erste Bildsignal entsprechend dem schmalbandigen Licht und das zweite Bildsignal entsprechend dem Fluoreszenzlicht.

3. Endoskopvorrichtung nach Anspruch 2,
bei der die Blutgefäßtiefeninformations-Berechnungseinheit eine Tiefeninformationstabelle enthält, die eine Korrelation zwischen einer Blutgefäßtiefe und einem Verhältnis zwischen dem ersten Bildsignal und dem G-Bildsignal aufzeichnet, wobei das Verhältnis in der Form $B_1/G$ ausgedrückt wird, und
wobei die Blutgefäßtiefeninformations-Berechnungseinheit die Blutgefäßtiefeninformation basierend auf dem $B_1/G$-Verhältnis und der Tiefeninformationstabelle berechnet.

4. Endoskopvorrichtung nach Anspruch 3,
bei der die Bildverarbeitungseinheit eine Blutgefäßtiefen-Bilderzeugungseinrichtung enthält, die basierend auf dem $B_1/G$-Verhältnis ein Blutgefäßtiefen-Bild erzeugt.

5. Endoskopvorrichtung nach einem der Ansprüche 1 bis 4,
bei der die Spektralabschätzinformation Matrixinformation ist, die verwendet wird zum Erzeugen eines Spektralbildsignals aus dem zweiten Bildsignal, und
wobei die Spektralabschätzinformation-Berechnungseinheit das Spektralbildsignal aus dem zweiten Bildsignal erzeugt, indem sie die Spektralabschätzinformation berechnet.

6. Endoskopvorrichtung nach Anspruch 5,
bei der die Bildverarbeitungseinheit eine Spektralbilderzeugungseinrichtung enthält, die eine Mehrzahl von Spektralbildern, die sich voneinander in Wellenlängenbandinformation unterscheiden, aus dem Spektralbildsignal berechnet.

7. Endoskopvorrichtung nach Anspruch 6,
bei der die mehreren Spektralbilder Spektralbilder sind, die verschiedene Wellenlängenbänder von 5 nm aufweisen.

8. Endoskopvorrichtung nach einem der Ansprüche 1 bis 7,
bei der die Lichtquelle eine blaue Laserlichtquelle mit einer mittleren Emissionswellenlänge von 445 nm ist.

9. Endoskopvorrichtung nach einem der Ansprüche 1 bis 8,
bei der die Verarbeitungseinrichtung weiterhin eine Normallicht-Betrachtungsbilderzeugungseinheit enthält, die basierend auf dem Bildsignal ein Normallicht-Betrachtungsbild erzeugt, und eine Anzeigevorrichtung enthält, welche ein von der Verarbeitungseinrichtung erzeugtes Bild anzeigt.

10. Endoskopvorrichtung nach Anspruch 9,
bei der eine Mehrzahl Anzeigevorrichtungen, jeweils identisch zu der Anzeigevorrichtung, die ein von der Verarbeitungseinrichtung erzeugtes Bild anzeigt, vorgesehen sind, wobei mindestens ein Speziallicht-Betrachtungsbild, welches das Blutgefäßtiefenbild und das Spektralbild enthält, auf mindestens einer der Anzeigevorrichtungen angezeigt wird, und das Normallicht-Betrachtungsbild auf mindestens einer der übrigen Anzeigevorrichtungen angezeigt wird.

11. Endoskopvorrichtung nach Anspruch 9 oder 10,

bei der die Verarbeitungseinrichtung eine Steuereinheit enthält, welche die Bildanzeige auf der Anzeigevorrichtung steuert,

wobei in der Verarbeitungseinrichtung mindestens zwei Anzeigemodi eingerichtet sind von einem Anzeigemodus, in welchem nur ein Normallicht-Betrachtungsbild auf der Anzeigevorrichtung angezeigt wird, einem Anzeigemodus, in welchem nur ein Speziallicht-Betrachtungsbild auf der Anzeigevorrichtung angezeigt wird, einem Anzeigemodus, in welchem sowohl ein Normallicht-Betrachtungsbild als auch ein Speziallicht-Betrachtungsbild oder beide verschiedenen Typen von Speziallicht-Betrachtungsbilder auf der Anzeigevorrichtung in ihrer Gesamtheit angezeigt werden, einem Anzeigemodus, in welchem sowohl ein Normallicht-Betrachtungsbild und ein Spezial-Betrachtungsbild oder beide verschiedene Typen von Speziallicht-Betrachtungsbildern angezeigt werden und der Anzeigebereich änderbar ist, und einem Anzeigemodus, in welchem ein Normallicht-Betrachtungsbild und ein Speziallicht-Betrachtungsbild oder zwei verschiedene Typen von Speziallicht-Betrachtungsbilder umgeschaltet und angezeigt werden, und wobei die Verarbeitungseinrichtung weiterhin eine Auswähleinrichtung aufweist, um eine Auswahl aus den Anzeigemodi vorzunehmen.

## Revendications

1. Endoscope (12) comprenant :

   un dispositif formant source de lumière (16) incluant une source de lumière (60) rayonnant une lumière à bande étroite ayant une largeur de bande de longueur d'onde prédéterminée rétrécie en relation avec les caractéristiques spectrales d'une structure et d'un constituant de corps vivant en tant que sujet, et une substance fluorescente (24) excitée par la lumière à bande étroite de façon à émettre une lumière fluorescente prédéterminée et qui rayonne la lumière d'éclairage incluant la lumière à bande étroite et la lumière fluorescente ;
   un corps d'endoscope qui rayonne la lumière d'éclairage vers le sujet et qui comporte un élément d'imagerie (48) capturant une image en utilisant la lumière renvoyée par le sujet de la lumière d'éclairage et fournissant en sortie un signal d'image ; et
   un dispositif de traitement (14) **caractérisé en ce qu'**il comporte une unité de division de signal (86) divisant le signal d'image en un premier signal d'image correspondant à la lumière à bande étroite et un second signal d'image correspondant à la lumière fluorescente, une unité de calcul d'informations de profondeur de vaisseau sanguin (88) calculant des informations de profondeur de vaisseau sanguin en se basant sur le premier signal d'image et le second signal d'image, une unité de calcul d'informations d'estimation spectrale (90) calculant des informations d'estimation spectrale en se basant sur le second signal d'image, et une unité de traitement d'image (92) générant une image capturée à partir du premier signal d'image, du second signal d'image, des informations de profondeur de vaisseau sanguin et des informations d'estimation spectrale.

2. Endoscope selon la revendication 1,
   dans lequel le signal d'image comporte un signal d'image B, un signal d'image G et un signal d'image R fournis en sortie en relation avec les caractéristiques de sensibilité spectrale de l'élément d'imagerie,
   dans lequel l'unité de division de signal comporte un moyen d'estimation de signal et un moyen de correction de calcul, et
   dans lequel le moyen d'estimation de signal estime le signal d'image B correspondant à la lumière fluorescente provenant du signal d'image G du signal d'image, et le moyen de correction de calcul divise le signal d'image B correspondant à la lumière fluorescente du signal d'image B du signal d'image, de façon que le signal d'image soit divisé par l'unité de division de signal en un premier signal d'image correspondant à la lumière à bande étroite et un second signal d'image correspondant à la lumière fluorescente.

3. Endoscope selon la revendication 2,
   dans lequel l'unité de calcul d'informations de profondeur de vaisseau sanguin comporte une table d'informations de profondeur contenant une corrélation entre la profondeur d'un vaisseau sanguin et le rapport entre le premier signal d'image et le signal d'image G, le rapport étant exprimé par $B_1/G$, et
   dans lequel l'unité de calcul d'informations de profondeur de vaisseau sanguin calcule les informations de profondeur de vaisseau sanguin en se basant sur le rapport $B_1/G$ et sur la table d'informations de profondeur.

4. Endoscope selon la revendication 3,
   dans lequel l'unité de traitement d'image comporte un moyen générateur d'image de profondeur de vaisseau sanguin qui génère une image de profondeur de vaisseau sanguin en se basant sur le rapport $B_1/G$.

**5.** Endoscope selon l'une quelconque des revendications 1 à 4, dans lequel les informations d'estimation spectrale sont des informations matricielles utilisées pour générer un signal d'image spectrale à partir du second signal d'image, et
dans lequel l'unité de calcul d'informations d'estimation spectrale génère le signal d'image spectrale d'après le second signal d'image en calculant les informations d'estimation spectrale.

**6.** Endoscope selon la revendication 5,
dans lequel l'unité de traitement d'image comporte un moyen générateur d'image spectrale qui génère à partir du signal d'image spectrale une pluralité d'images spectrales dont les informations de bande de longueur d'onde sont différentes les unes des autres.

**7.** Endoscope selon la revendication 6,
dans lequel la pluralité d'images spectrales sont des images spectrales ayant des bandes de longueur d'onde qui diffèrent de 5 nm.

**8.** Endoscope selon l'une quelconque des revendications 1 à 7,
dans lequel la source de lumière est une source de lumière laser bleue ayant une longueur d'onde d'émission centrale de 445 nm.

**9.** Endoscope selon l'une quelconque des revendications 1 à 8,
dans lequel le dispositif de traitement comporte en outre une unité génératrice d'image d'observation de lumière normale qui génère une image d'observation de lumière normale en se basant sur le signal d'image, et un dispositif d'affichage qui affiche une image générée par le dispositif de traitement sur celui-ci.

**10.** Endoscope selon la revendication 9,
dans lequel une pluralité de dispositifs d'affichage sont prévus, chacun étant identique au dispositif d'affichage qui affiche une image générée par le dispositif de traitement sur celui-ci, au moins une image d'observation de lumière spéciale incluant l'image de profondeur de vaisseau sanguin et l'image spectrale est affichée sur au moins l'un des dispositifs d'affichage, et l'image d'observation de lumière normale est affichée sur au moins l'un des dispositifs d'affichages restants.

**11.** Endoscope selon la revendication 9 ou 10,
dans lequel le dispositif de traitement comporte une unité de commande qui commande l'affichage d'image sur le dispositif d'affichage,
dans lequel, dans le dispositif de traitement, au moins deux modes d'affichages différents sont déterminés d'après un mode d'affichage dans lequel seule une image d'observation de lumière normale est affichée sur le dispositif d'affichage, un mode d'affichage dans lequel seul une d'image d'observation de lumière spéciale est affichée sur le dispositif d'affichage, un mode d'affichage dans lequel à la fois une image d'observation de lumière normale et une image d'observation de lumière spéciale, ou les deux types différents d'images d'observation de lumière spéciale sont affichés sur le dispositif d'affichage dans leur totalité, un mode d'affichage dans lequel à la fois une image d'observation de lumière normale et une image d'observation de lumière spéciale, ou les deux types différents d'images d'observation de lumière spéciale sont affichés et la plage d'affichage est modifiable, et un mode d'affichage dans lequel une image d'observation de lumière normale et une image d'observation de lumière spéciale ou deux types différents d'images d'observation de lumière spéciale sont commutés et affichés, et
dans lequel le dispositif de traitement comporte en outre un moyen de sélection pour effectuer une sélection à partir des modes d'affichage.

# FIG. 1

FIG. 2

EP 2 505 121 B1

# FIG. 3

48a

| R | G | R | G |
|---|---|---|---|
| R | B | R | B |
| R | G | R | G |
| R | B | R | B |

# FIG. 4

445nm LASER LIGHT

FLUORESCENT LIGHT OF
FLUORESCENT SUBSTANCE

EMISSION INTENSITY

405 445    500              600              700

WAVELENGTH(nm)

# FIG. 5

# FIG. 6

# FIG. 7

48a COLOR FILTER — 14a

IMAGE SIGNAL PROCESSING SECTION

ENDOSCOPE 12

- SENSOR 48
- AFE 56

76 — DSP

78 — STORAGE UNIT

ORDINARY LIGHT IMAGE GENERATING UNIT — 80
- 80a — GAIN ADJUSTING SUB-UNIT
- IMAGE PROCESSING SUB-UNIT — 80b

84 — DISPLAY SIGNAL GENERATING UNIT

18 — DISPLAY DEVICE

20 — INPUT DEVICE

SPECIAL LIGHT IMAGE GENERATING UNIT — 82

88 — BLOOD VESSEL DEPTH INFORMATION CALCULATING UNIT
- DEPTH INFORMATION TABLE — 88a

86 — SIGNAL DIVIDING UNIT
- 86a — SIGNAL ESTIMATING SUB-UNIT
- 86b — CALCULATION CORRECTION SUB-UNIT

90 — SPECTRAL ESTIMATION INFORMATION CALCULATING UNIT
- MATRIX CALCULATING SUB-UNIT — 90a
- IMAGE SIGNAL CORRECTING SUB-UNIT — 90b

92 — IMAGE PROCESSING UNIT
- BLOOD VESSEL DEPTH IMAGE GENERATING SUB-UNIT — 92a
- SPECTRAL IMAGE GENERATING SUB-UNIT — 92b

EP 2 505 121 B1

FIG. 8A

x "SPECTRAL
    REFLECTIVITY
    OF LIVING BODY"

x "COLOR FILTER
    CHARACTERISTICS
    OF CCD SENSOR"

B-LIGHT      G-LIGHT      R-LIGHT
COMPONENT COMPONENT COMPONENT

FIG. 8B

x "SPECTRAL
    REFLECTIVITY
    OF LIVING BODY"

x "COLOR FILTER
    CHARACTERISTICS
    OF CCD SENSOR"

B-LIGHT      G-LIGHT      R-LIGHT
COMPONENT COMPONENT COMPONENT

FIG. 8C

x "SPECTRAL
    REFLECTIVITY
    OF LIVING BODY"

x "COLOR FILTER
    CHARACTERISTICS
    OF CCD SENSOR"

B-LIGHT      G-LIGHT      R-LIGHT
COMPONENT COMPONENT COMPONENT

# FIG. 9

# FIG. 10

**EP 2 505 121 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4504324 B

- US 2011071352 A1